# EUROPÄISCHE PATENTSCHRIFT

(11) **EP 2 682 149 B1**
(45) Veröffentlichungstag und Bekanntmachung des Hinweises auf die Patenterteilung: **06.03.2019**
(21) Anmeldenummer: 13002720.4
(22) Anmeldetag: 24.05.2013
(51) Int. Cl.: A61M 16/06, A61M 16/00, A61M 16/08, A61M 16/20

(54) **Sauerstoffgerät sowie Atemluftschlauch und Brandschutzmodul dafür**
Oxygen device and breathing air hose and fire safety module therefor
Appareil à oxygène ainsi que flexible d'air de respiration et module anti-incendie correspondant

(30) Priorität: 02.07.2012 DE 102012013055
(43) Veröffentlichungstag der Anmeldung: 08.01.2014
(73) Patentinhaber: LUCHS Medizin GmbH & Co. KG, 31177 Harsum (DE)
(72) Erfinder: Brey, Jörg, 31787 Hameln (DE); Hoya, Stefan, 31177 Harsum (DE)
(74) Vertreter: Plöger, Jan Manfred

(56) Entgegenhaltungen:
- EP-A2- 1 014 527
- WO-A1-2008/068508
- WO-A1-2012/001951
- GB-A- 2 417 425
- US-A- 2 399 843
- US-A- 3 070 132
- US-A- 3 580 983
- US-A- 3 721 898
- US-A- 5 156 180
- US-A- 5 927 316
- US-A1- 2009 288 662
- US-A1- 2010 043 912
- US-A1- 2010 300 708

## Beschreibung

Die Erfindung betrifft ein Sauerstoffgerät zum Abgeben eines sauerstoffangereicherten Atemgases mit (a) einer Atemgasquelle, die zum Erzeugen des sauerstoffangereicherten Atemgases eingerichtet ist, und (b) einem Atemluftschlauch, der ausgebildet zum Leiten des Atemgases von der Atemgasquelle zu einem Verbrauchsort. Gemäß einem zweiten Aspekt betrifft die Erfindung einen Atemluftschlauch für ein Sauerstoffgerät, mit (i) einem Kopfabschnitt, der zum Befestigen an einem Kopf eines Patienten ausgebildet ist, und (ii) einem Nasenteil, das zum Abgeben des Atemgases in die Nase des Patienten ausgebildet ist. Gemäß einem weiteren Aspekt betrifft die Erfindung ein Brandschutzmodul.

Derartige Sauerstoffgeräte werden für Patienten mit Lungenschwäche eingesetzt und sind mobil, so dass sie auch in einer Wohnung eines Patienten eingesetzt oder gar mitgeführt werden können. Insbesondere werden Sauerstoffgeräte von den Patienten auch im Bett benutzt. Ein Konzentrator für ein solches Sauerstoffgerät ist in der DE 25 24 242 A1 beschrieben.

Trotz entsprechender Warnungen rauchen Patienten, während das Sauerstoffgerät benutzt wird. Durch die sehr hohe Sauerstoffkonzentration im Atemgas kann es zu Bränden kommen. Da der Atemluftschlauch ein Wegwerfprodukt ist, wird er meist aus PVC hergestellt und kann aufgrund der hohen Sauerstoffkonzentration des Atemgases selbst Feuer fangen. Das bedeutet eine hohe Brandgefahr und unter Umständen Lebensgefahr für den Patienten.

Es ist bekannt, in den Atemluftschlauch eine Flammendurchtrittssperre einzubauen, die ein Übergreifen des Brandes von der einen Seite auf die gegenüberliegende Seite unterbindet.

Aus der US 2010/0300708 A1 ist ein System zur Branderkennung in Endotrachealtuben für chirurgische Operationen bekannt. In den Tuben sind Thermowiderstände angeordnet, sodass aus einer Widerstandsänderung auf eine Temperaturänderung geschlossen werden kann. Wird ein Temperatur-Schwellenwert überschritten, wird der Brand durch Bespritzen mit Salzwasser oder durch Ausblasen gelöscht. Ein derartiges System ist aufwändig und außerhalb eines Operationssaals kaum einsetzbar

Die WO 2012/001951 A1 beschreibt mehrere Systeme zur Temperaturmessung in einem Sauerstoffgerät. Es ist das Ziel, ein Übergreifen eines Brands auf das Sauerstoffgerät selbst zu vermeiden. Das erste System enthält einen Temperatursensor und es existiert eine vorgebbare Temperatur, oberhalb derer die Sauerstoffzufuhr unterbrochen wird. Ein dort beschriebenes System betrifft die Überwachung der Temperatur des Atemluftschlauchs. Raucht der Patient und entzündet sich der Schlauch, so breitet sich der Brand durch den Schlauch aus und erreicht ein Koppelstück, das verbrennt. Es wird die Temperatur oder die Temperaturanstiegsgeschwindigkeit ermittelt und die Sauerstoffzufuhr unterbrochen, wenn beispielsweise 40°C überschritten werden oder die Temperatur für mehr als 3 Sekunden um mehr als 1°C/s steigt. Zudem wird vorgeschlagen, dass zwei Drähte im Atemluftschlauch einen Flammenstab bilden. Dieses System schlägt nur dann an, wenn eine Flamme in unmittelbarer Nähe der Drähte existiert.

Zudem wird ein drahtförmiges Thermoelement aus zwei verdrillten Drähten vorgeschlagen, die gegeneinander isoliert sind. Im Brandfall schmilzt die Isolation und es entsteht eine Thermospannung, die detektiert wird. Um der hohen Flammentemperatur standzuhalten, werden Alumel und Chromel, Platin, Rhodium und Iridium als Drahtmaterialbestandteile vorgeschlagen. Nachteilig an diesem System sind die aufwändige Herstellung und das Risiko, dass im Brandfall keine auswertbare Thermospannung messbar ist.

Der Erfindung liegt die Aufgabe zugrunde, die Brandsicherheit mit einfachen Mitteln zu verbessern.

Die Erfindung löst das Problem durch ein Sauerstoffgerät mit den Merkmalen von Anspruch 1.

Vorteilhaft an der Erfindung ist, dass die Zufuhr von Sauerstoff in den Atemluftschlauch bereits dann unterbindbar ist, wenn der Brand gerade erst begonnen hat. Die Unterbrechung der Zufuhr an Atemgas bewirkt ein sofortiges Löschen der Flamme. Das ist insofern überraschend, als im Atemluftschlauch weiterhin Sauerstoff vorhanden ist. Es hat sich jedoch herausgestellt, dass der lokale Brandherd so viel Sauerstoff verbraucht, dass die Sauerstoffkonzentration in einer Umgebung des Brandherdes rasch auf einen Wert abfällt, unterhalb dem der Atemluftschlauch nicht mehr weiter brennt. Der Brand erlischt daher nach so kurzer Zeit, dass eine Ausbreitung auf die Umgebung, beispielsweise auf ein Bett, in dem der Patient liegt, so gut wie ausgeschlossen ist. Es ist daher möglich, den Atemluftschlauch auf dem Bett liegend zu lagern, ohne dass wie bei bekannten Systemen befürchtet werden muss, dass das Bett Feuer fängt, bevor der Atemluftschlauch gelöscht werden kann.

Es ist ein weiterer Vorteil, dass der geschilderte Gewinn an Sicherheit mit technisch einfachen Mitteln erreicht werden kann. So ist es einfach möglich, den Atemluftschlauch mit eingelassenem Detektordraht herzustellen. So kann der Atemluftschlauch, wie in einer bevorzugten Ausführungsform vorgesehen, durch Umspritzen des Detektordrahts und damit in einem Extrusionsverfahren hergestellt werden. Ein derartiges Verfahren ist mit geringem technischem Aufwand durchführbar. Dem signifikanten Gewinn an Patientensicherheit steht damit nur ein geringer zusätzlicher Aufwand bei der Herstellung des Atemluftschlauchs gegenüber.

Ein weiterer Vorteil ist, dass der in dem Atemluftschlauch eingelassene Detektordraht nicht zu einer Erhöhung des Strömungswiderstandes des Atemgases führt. Anders als bei bekannten Flammensperren kann der Atemluftschlauch die gleichen Innen- und Außenabmessungen haben wie bei herkömmlichen Sauerstoffgeräten, ohne dass der Volumenstrom an Atemgas verringert oder ein erhöhter Gasdruck angelegt werden muss. Es ist daher möglich, den Atemluftschlauch mit Detektordraht mit bestehenden Sauerstoffgeräten zu verwenden.

Im Rahmen der vorliegenden Beschreibung wird unter der Atemgasquelle insbesondere eine Vorrichtung verstanden, die zum Abgeben eines sauerstoffreichen Atemgases ausgebildet ist. Insbesondere kann die Atemgasquelle einen Konzentrator umfassen, mittels dem das sauerstoffangereicherte Atemgas aus der Umgebungsluft herstellbar ist. Insbesondere umfasst der Konzentrator eine Membran, die auch als Molekularsieb bezeichnet werden kann, durch die Sauerstoff mit einer anderen Diffusionsgeschwindigkeit durchtritt als Stickstoff und gegebenenfalls weitere Luftbestandteile. Durch eine Hintereinanderschaltung derartiger Membranen (Molekularsieb) kann daher ein Atemgas mit deutlich erhöhtem Sauerstoffgehalt erhalten werden. Vorzugsweise beträgt der Sauerstoffgehalt des Atemgases zumindest 90 %. Alternativ oder zusätzlich kann die Atemgasquelle auch eine Gasflasche mit gasförmigem oder einen Thermobehälter mit einem flüssigen Sauerstoff umfassen. Eine derartige Atemgasquelle wird auch Flüssigsauerstoffgerät genannt.

Unter dem Atemluftschlauch wird insbesondere ein Kunststoffschlauch verstanden, der flexibel ist und an einem Ende einen Anschluss zum Einspeisen von Atemgas aufweist. An dem gegenüberliegenden Ende besitzt der Atemluftschlauch vorzugsweise eine sogenannte Nasenbrille. Eine Nasenbrille ist eine Vorrichtung, die hinter den Ohren eines Patienten befestigbar ist und im Bereich der Nasenlöcher des Patienten Austrittsöffnungen für das Atemgas aufweist.

Unter dem Detektordraht wird insbesondere ein langgestreckter elektrischer Leiter verstanden, der bei Hitzeeinwirkung schmilzt. Die genaue Struktur des Detektordrahts ist von untergeordneter Bedeutung. Es ist beispielsweise möglich, dass der Detektordraht helikal gewendelt ist, in Schlangenlinien verläuft oder gerade entlang einer Längserstreckung des Atemluftschlauchs verläuft. Der Detektordraht kann einadrig sein oder zumindest zwei Adern aufweisen, die vorzugsweise gegeneinander isoliert sind. Insbesondere ist der Detektordraht biegealterungsfest, das heißt, dass ein Biegen des Atemluftschlauchs nicht zu einem Zerstören des Detektordrahts führt. Unter dem Biegen des Atemluftschlauchs wird jedoch kein Knicken verstanden. Zwar ist es vorteilhaft, wenn der Detektordraht auch bei einem Knicken des Atemluftschlauchs nicht bricht, das ist aber nicht notwendig, da der Atemluftschlauch in aller Regel nicht geknickt wird. Ein Knicken des Atemluftschlauchs würde nämlich die Zufuhr des Atemgases unterbinden.

Gemäß einer bevorzugten Ausführungsform umfasst das Brandschutzmodul ein Umlenk-Ventil, das mit der Atemgasquelle und dem Atemluftschlauch verbunden ist und so mit dem Detektordraht verschaltet ist, dass das Ventil, insbesondere ein Umlenk-Ventil nur dann Atemgas in den Atemluftschlauch abgibt, wenn der Detektordraht nicht durch einen brennenden Atemluftschlauch beeinflusst ist.

Unter dem Umlenk-Ventil wird insbesondere jede Vorrichtung verstanden, die eine Eingangsseite hat, durch die das Atemgas einströmt, und die einen Auslass hat, der in einer ersten Schaltstellung mit dem Einlass verbunden ist und der zum Atemluftschlauch führt, wobei das Umlenk-Ventil in eine zweite Stellung bringbar ist, in der die Zufuhr von Atemgas zum Atemluftschlauch unterbunden ist.

Es ist vorteilhaft, nicht aber notwendig, dass das Umlenk-Ventil einen zweiten Ausfass besitzt, durch den das Atemgas in eine Umgebung abgegeben wird. Das hat den Vorteil, dass die Atemgasquelle nie gegen einen signifikant erhöhten Widerstand arbeiten muss. Vorteilhaft daran ist, dass bestehende Sauerstoffgeräte mit einem derartigen Brandschutzmodul-Umlenk-Ventil nachgerüstet werden können.

Erfindungsgemäß besteht der Detektordraht aus einem Detektordrahtmaterial, das einen Schmelzpunkt hat, wobei das Schmelzdrahtmaterial so gewählt ist, dass der Detektordraht zumindest lokal schmilzt, wenn der Atemluftschlauch brennt. Insbesondere beträgt der Schmelzpunkt des Detektordrahts höchstens 390°C (Kupferlackdraht), Das Durchschmelzen des Schmelzdrahts ist besonders einfach detektierbar. Das Sauerstoffgerät ist daher robust und betriebssicher betreibbar.

Erfindungsgemäß ist es vorteilhaft, wenn das Sauerstoffgerät Atemgas in den Atemluftschlauch abgibt, solange der Detektordraht unversehrt ist. In anderen Worten ist das Sauerstoffgerät so eingerichtet, dass die Abgabe von Atemgas unabhängig von einer Temperatur des Detektordrahts ist, solange dieser unversehrt ist. Anders ausgedrückt ist das Sauerstoffgerät ausgebildet zum Unterbrechen des Abgebens des Atemgases in den Atemluftschlauch, wenn der Detektordraht lokal zerstört ist, insbesondere wenn der elektrische Widerstand des Detektordrahts - im Rahmen der Messgenauigkeit - unendlich geworden ist.
Vorzugsweise ist ein Sauerstoffgerät ein Gerät für die häusliche Sauerstofftherapie. Ein derartiges Gerät ist auch von einem Patienten bewegbar. Es gibt einen konstanten Strom an Sauerstoff ab und unterscheidet sich dadurch von einem Beatmungsgerät, das einen pulsierenden Atemgasstrom abgibt. In anderen Worten gibt das Sauerstoffgerät - vorzugsweise - anders als ein Beatmungsgerät das Atemgas mit einem zeitlich zumindest im Wesentlichen konstanten Druck ab, beispielsweise schwankt der Druck innerhalb einer Sekunde um weniger als 10 hPa.Gemäß einer bevorzugten Ausführungsform ist der Detektordraht in eine Wandung des Atemluftschlauchs einextrudiert. In anderen Worten ist der Atemluftschlauch fügestellenfrei und umgibt den Detektordraht, wobei der Detektordraht über seine gesamte Mantelfläche in Kontakt mit dem Wandungsmaterial steht, also dem Material, aus dem der Schlauch abgesehen vom Draht hergestellt ist.

Gemäß einer bevorzugten Ausführungsform umfasst das Sauerstoffgerät eine elektrische Steuereinheit, die eingerichtet ist zum automatischen Durchführen eines Verfahrens mit den Schritten (i) Erfassen, ob sich die elektrische Eigenschaft des Detektordrahts so verändert, dass auf einen Brand des Atemluftschlauchs geschlossen werden kann, und bejahendenfalls (ii) Abgeben eines Warnsignals, insbesondere eines Notrufsignals. Selbstverständlich erfolgt verneinendenfalls keine Abgabe des Warnsignals. Insbesondere ist die elektrische Steuereinheit eingerichtet zum Erfassen, ob der Detektordraht zerstört ist, indem der elektrische Widerstand des Detektordrahts gemessen und mit einem Schwellenwert verglichen wird. Wenn der Detektordraht ein Schmelzdraht ist, so steigt der elektrische Widerstand bei einem lokalen Schmelzen um mehrere Größenordnungen an, was einfach detektiert werden kann. Das Notsignal kann auf eine sogenannte Schwesternrufanlage/Alarmbereitschaft geschaltet werden. Möglich ist es, dass das Notrufsignal per Mobilfunk abgesetzt wird, beispielsweise durch automatischen Anruf oder eine Kurznachricht.

Gemäß einer bevorzugten Ausführungsform ist das Ventil ein Elektroventil. In anderen Worten umfasst das Ventil einen Elektromagneten, der auf einen Ventilkörper des Ventils einwirkt. Alternativ ist es aber auch möglich, dass das Ventil pneumatisch betätigt wird.

Gemäß einer bevorzugten Ausführungsform ist die elektrische Steuereinheit eingerichtet zum automatischen Erfassen, ob der Schmelzdraht ununterbrochen ist und bejahendenfalls Bringen oder Halten des Ventils in einer Stellung, in der Atemgas in den Atemluftschlauch abgegeben wird. Hierzu umfasst die Steuereinheit eine Messvorrichtung zum Erfassen einer elektrischen Größe, insbesondere Strom, Spannung oder Widerstand, der in eindeutigem Zusammenhang mit dem elektrischen Widerstand des Detektordrahts steht. Erhöht sich der elektrische Widerstand um zumindest den Faktor 100, insbesondere 1000, wird die Zufuhr des Atemgases in den Atemluftschlauch unterbrochen. Geringere Schwankungen des elektrischen Widerstands, insbesondere ein Sinken des Widerstands, sind irrelevant. Die Feststellung der Zerstörung des Detektordrahts ist besonders einfach und prozesssicher durchführbar. Insbesondere ist der Detektordraht kein Widerstandsdraht oder Thermoelement, seine Temperatur ist irrelevant.

Ein erfindungsgemäßer Atemluftschlauch besitzt gemäß einer bevorzugten Ausführungsform eine elektrische Kontaktierung, so dass der Detektordraht mittels eines Verbinders von außen kontaktierbar ist. In anderen Worten besitzt der Atemluftschlauch zumindest zwei elektrische Kontakte, über die ein elektrischer Strom durch den Detektordraht einprägbar ist. Vorzugsweise umfasst die Kontaktierung ein Metallrohr. Das Einstecken des Metallrohrs in eine Abgabeöffnung des Konzentrators oder des Brandschutzmoduls reicht, um den Detektordraht elektrisch zu kontaktieren und gleichzeitig die Verbindung für den Atemluftstrom herzustellen.

Günstig ist es, wenn der Atemluftschlauch zwei Teil-Schläuche aufweist, die beidseits am Nasenteil befestigt sind. Die Teil-Schläuche enthalten jeweils einen Schmelzdraht. Die Schmelzdrähte beider Teil-Schläuche werden beispielsweise durch einen elektrischen Leiter elektrisch miteinander verbunden, der lose im Nasenteil liegt oder in dieses einextrudiert ist.

Vorzugsweise ist der Atemluftschlauch in einer Atmosphäre mit höchstens 25% Sauerstoff selbstlöschend. Das heißt, dass ein Brand des Atemluftschlauchs ohne externes Zutun erlischt, wenn sich die Sauerstoffkonzentration der Erdatmosphäre einstellt. Das Erlöschen erfolgt in aller Regel so schnell, dass ein Übergreifen des Brands auf andere Objekte wie Bettlaken nicht zu befürchten ist.

Im Folgenden wird die Erfindung anhand der beigefügten Zeichnungen näher erläutert. Dabei zeigt
- Figur 1: eine schematische Ansicht eines erfindungsgemäßen Atemgeräts, eines erfindungsgemäßen Atemluftschlauchs und eines erfindungsgemäßen Brandschutzmoduls.
- Figur 2: zeigt einen erfindungsgemäßen Atemluftschlauch gemäß einer zweiten Ausführungsform.

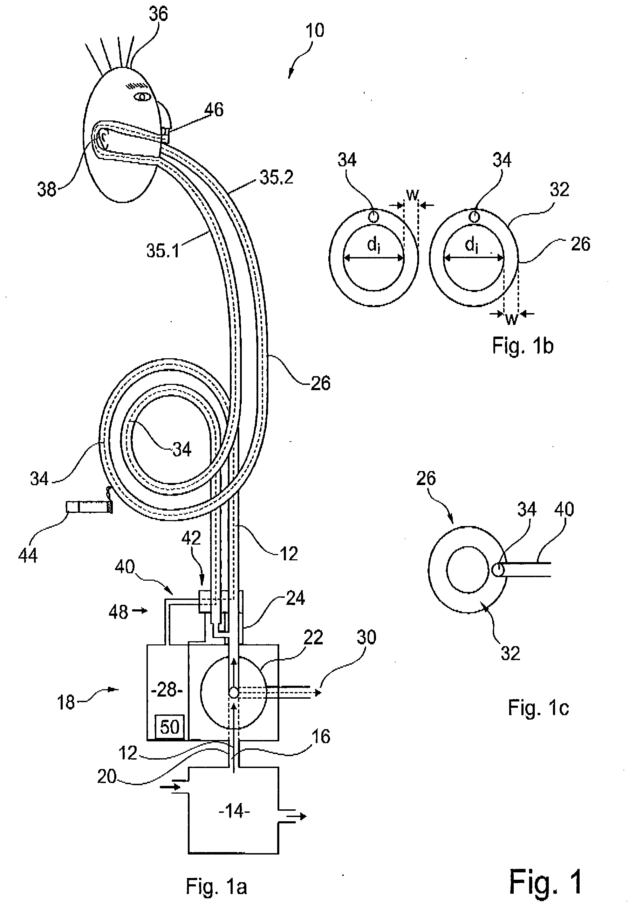

Figur 1 zeigt in der Teil-Figur 1a ein erfindungsgemäßes Sauerstoffgerät 10 zum Abgeben eines sauerstoffangereicherten Atemgases 12 mit über 80%, insbesondere über 90 %, Sauerstoff, das von einer Atemgasquelle 14 aus Umgebungsluft erzeugt wird. Die Atemgasquelle 14 besitzt eine Abgabeöffnung 16, durch die Atemgas 12 abgegeben wird.

Das Sauerstoffgerät 10 umfasst ein Brandschutzmodul 18, das von dem Grundkörper der Atemgasquelle 14 zerstörungsfrei getrennt werden kann und eine Eingangsöffnung 20 aufweist, die mit der Abgabeöffnung 16 so verbunden ist, so dass das Atemgas einströmen kann. Die Eingangsöffnung 20 steht in Verbindung mit einem Ventil 22, das in der Teil-Figur 1a in seiner Durchgangs-Schaltstellung gezeigt ist. In dieser Durchgangs-Schaltstellung verbindet das Ventil 22 die Eingangsöffnung 20 mit einem Schlauchanschluss 24, an den ein Atemgasschlauch 26 angeschlossen ist.

Das Ventil 22 ist ein Elektroventil und wird von einer elektrischen Steuereinheit 28 angesteuert. Das Ventil 22 besitzt eine Entlüftungs-Schaltstellung, in der die Eingangsöffnung 20 mit einer Ablassöffnung 30 verbunden ist. Die Ablassöffnung 30 führt in die Umgebung, so dass das Atemgas 12 in der Entlüftungs-Schaltstellung in die Umgebung entweicht und nicht in den Atemgasschlauch 26 gelangt. Der Strom an Atemgas in der Entlüftungs-Schaltstellung ist durch einen gestrichelten Pfeil eingezeichnet, der Gasstrom in der Durchgangs-Schaltstellung mit einem durchgehenden Pfeil.

Der Atemgasschlauch 26 hat, wie in der Teil-Figur 1b gezeigt, einen Innendurchmesser dᵢ, der in der Regel zwischen 2 und 5 mm liegt. Eine Wandstärke w liegt in der Regel zwischen 1 und 2 mm. Der Atemgasschlauch 26 ist aus PVC gefertigt.

In eine Wandung 32 (vgl. Figur 1b) ist ein Detektordraht 34 eingelassen, der aus Kupferlackdraht besteht. Der Detektordraht hat einen Schmelzpunkt von T_{Schmelz} = 390 °C. Oberhalb dieser Temperatur schmilzt der Detektordraht 34 lokal und/oder wird lokal zerstört. Dadurch bricht seine Fähigkeit, den elektrischen Strom zu leiten, zusammen.

Der Atemluftschlauch 26 ist zweisträngig ausgebildet, das heißt, dass das Atemgas 12 in zwei parallel zueinander verlaufenden Röhren 35.1, 35.2 zu einem Kopf 36 eines Patienten strömt, wobei die beiden Röhren 35.1, 35.2 miteinander verbunden sind und sich in einer Nasenbrille 38 treffen Die Nasenbrille 38 besitzt ein Nasenstück 46, durch das das Atemgas direkt in eine Nase des Patienten abgegeben wird.

Alternativ ist der Atemluftschlauch 26 einlumig ausgebildet. In diesem Fall kann der Detektordraht 34 zumindest zwei Adern aufweisen, das heißt, dass der der elektrische Strom durch eine der Adern von der Atemgasquelle 14 auf die Nasebrille 38 zu fließt und in einer anderen Ader von der Nasenbrille 38 zur Atemgasquelle 14. Beispielsweise sind die beiden Adern miteinander verdrillt und gegeneinander durch eine Lackschicht elektrisch isoliert.

Der Atemluftschlauch 26 besitzt, wie in Teil-Figur 1c dargestellt, eine elektrische Kontaktierung 40, die dadurch gebildet ist, dass der Detektordraht 34 benachbart zum Brandschutzmodul 18 durch die Wandung 32 nach außen hinausgeführt ist. Das Brandschutzmodul 18 besitzt einen Verbinder 42, mittels dem die Kontaktierung 40 elektrisch mit der Steuereinheit 28 verbunden ist. Im Betrieb legt die Steuereinheit 28 über die beiden elektrischen Pole der Kontaktierung 40 eine elektrische Spannung an, so dass ein elektrischer Strom entlang der gesamten Längserstreckung des Atemgasschlauchs 26 fließt.

Wird, beispielsweise von einer Zigarette 44, der Atemluftschlauch 26 entzündet und der Detektordraht 34 lokal zerstört, so ist der Stromkreis sofort unterbrochen und der elektrische Widerstand R, der von der Steuereinheit 28 gemessen wird, steigt um zumindest eine Größenordnung an. In diesem Fall steuert die Steuereinheit 28 das Ventil 22 so an, dass es in die Entlüftungs-Schaltstellung kommt und das sauerstoffreiche Atemgas in die Umgebung ablässt. Der Brand erlischt dann selbsttätig.

Der Atemluftschlauch 26 ist über eine lösbare Verbindung 48, beispielsweise eine Schraub- und/oder Steckverbindung mit dem Brandschutzmodul 18 verbunden. Der Verbinder 42 ist so angeordnet, dass er die Kontaktierung 40 dann kontaktiert, wenn der Atemluftschlauch 26 mit dem Schlauchanschluss 24 verbunden wird.

Detektiert die Steuereinheit 28 einen Brand, so sendet sie zum Beispiel über ein schematisch eingezeichnetes Mobilfunkmodul eine Notfallnachricht, indem das Mobilfunkmodul 50 eine eingespeicherte Nummer anruft und dort eine Nachricht hinterlässt, dass ein Brand detektiert und gelöscht wurde. Alternativ oder zusätzlich kann eine Kurznachricht (SMS) gesendet werden.

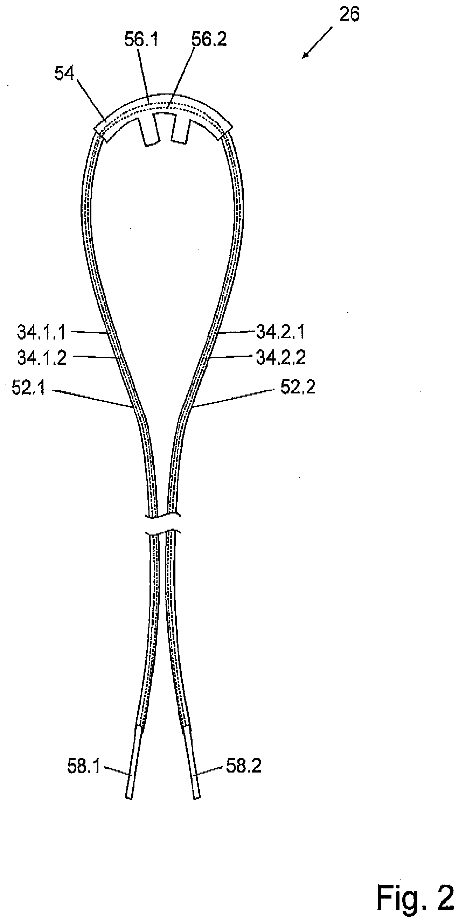

Figur 2 zeigt einen Atemluftschlauch 26, der zwei Teil-Schläuche 52.1, 52.2 aufweist, die jeweils mit einem Nasenteil 54 verbunden sind.

Das Nasenteil 54 ist ein extrudiertes Kunststoffteil, das einen ersten elektrischen Leiter 56.1 und einen zweiten elektrischen Leiter 56.1 und einen zweiten elektrischen Leiter 56.2 umfasst. Diese liegen in der vorliegenden Ausführungsform lose in einem Innenraum des Nasenteils 54. Es ist aber auch möglich, dass die elektrischen Leiter einextrudiert sind.

Der erste Teilschlauch 52.1 umfasst einen ersten Teil-Schmelzdraht 34.1 und einen zweiten Teil-Schmelzdraht 34.1.2, die voneinander beabstandet und daher gegeneinander elektrisch isoliert in einer Wandung des Teil-Schlauchs angeordnet sind. Der zweite Teil-Schlauch 34.2 ist gleich aufgebaut und besitzt zumindest einen zweiten Teil-Schmelzdraht 34.2.1 und im vorliegenden Fall einen weiteren Teil-Schmelzdraht 34.2.2. Die beiden Teil-Schmelzdrähte unterschiedlicher Teil-Schläuche sind durch jeweils einen elektrischen Leiter 56 verbunden. So sind die Teil-Schmelzdrähte 34.1.1 und 34.2.1 durch den elektrischen Leiter 56.1 miteinander verbunden. Zur Herstellung werden die Teil-Schmelzdrähte ein kurzes Stück, beispielsweise 1 cm, am jeweiligen Ende freigelegt und kontaktiert, beispielsweise mit Lot oder Gleitlack.

An dem dem Nasenteil 54 abgewandten Ende weist der Atemluftschlauch 26 zwei Metallrohre 58.1, 58.2 auf, die jeweils mit allen Teil-Schmelzdrähten oder nur denjenigen Teil-Schmelzdrähten, die miteinander verbunden sind, elektrisch kontaktiert sind. Es ist auch möglich, dass die Metallrohre 58.1, 58.2 zwei gegeneinander isolierte metallische Abschnitte haben, so dass jeweils ein Teil-Schmelzdraht mit einem dieser metallisch leitenden Bereiche verbunden werden kann.

Die Metallrohre werden in eine Ausgabeöffnung des Sauerstoffgerätes oder eines erfindungsgemäßen Brandschutzmoduls gesteckt. Durch sie fließt das Atemgas hindurch zum Nasenteil. Gleichzeitig werden die Schmelzdrähte elektrisch kontaktiert. Der Vorteil an der Verwendung von zwei gegeneinander isolierten Teil-Schmelzdrähten ist, dass es unbeachtlich ist, wenn bei der Herstellung einer der Schmelzdrähte beschädigt wird. Bei dem in Figur 2 gezeigten Aufbau ist es jedoch ausreichend und der Einfachheit halber günstiger, nur einen Teil-Schmelzdraht pro Teil-Schlauch zu verwenden.

**Bezugszeichenliste**

| | | | |
|---|---|---|---|
| 10 | Sauerstoffgerät | 50 | Mobilfunkmodul |
| 12 | Atemgas | 52 | Teil-Schlauch |
| 14 | Atemgasquelle | 54 | Nasenteil |
| 16 | Abgabeöffnung | 56 | Leiter |
| 18 | Brandschutzmodul | 58 | Metallrohr |
| 20 | Eingangsöffnung | d | Innendurchmesser |
| | | | |
| 22 | Ventil | w | Wandstärke |
| 24 | Schlauchanschluss | T_{Schmelz} | Schmelzpunkt |
| 26 | Atemluftschlauch | R | elektrischer Widerstand |
| 28 | Steuereinheit | | |
| | | | |
| 30 | Ablassöffnung | | |
| 32 | Wandung | | |
| 34 | Detektordraht | | |
| 35 | Röhre | | |
| 36 | Kopf | | |
| 38 | Nasenbrille | | |
| | | | |
| 40 | Kontaktierung | | |
| 42 | Verbinder | | |
| 44 | Zigarette | | |
| 46 | Nasenstück | | |
| 48 | Verbindung | | |

## Patentansprüche

1. Sauerstoffgerät zum Abgeben eines sauerstoffangereicherten Atemgases (12) mit
(a) einer Atemgasquelle (14), die zum Erzeugen des sauerstoffangereicherten Atemgases (12) eingerichtet ist, und
(b) einem Atemluftschlauch (26), der ausgebildet zum Leiten des Atemgases (12) von der Atemgasquelle (14) zu einem Verbrauchsort (36),
**dadurch gekennzeichnet, dass**
(c) der Atemluftschlauch (26) einen Detektordraht (34) aufweist, der als Schmelzdraht ausgebildet ist und aus einem Detektordrahtmaterial besteht, das so gewählt ist, dass der Detektordraht (34) zumindest lokal schmilzt, wenn der Atemluftschlauch (26) brennt, sodass der Detektordraht (34) lokal zerstört wird und der elektrische Widerstand (R) um zumindest eine Größenordnung ansteigt, und
(d) das Sauerstoffgerät (10) so ausgebildet ist, dass die Atemgasquelle (14) nur dann Atemgas (12) in den Atemluftschlauch (26) abgibt, wenn der Detektordraht (34) unversehrt ist und ausgebildet ist zum Unterbrechen des Abgebens des Atemgases in den Atemluftschlauch, wenn der Detektordraht lokal zerstört ist.

2. Sauerstoffgerät nach Anspruch 1, **gekennzeichnet durch** ein Ventil (22), insbesondere ein Umlenkventil,
- das mit der Atemgasquelle (14) und dem Atemluftschlauch (26) verbunden ist und
- so mit dem Detektordraht (34) verschaltet ist, dass das Ventil (22) nur dann Atemgas (12) in den Atemluftschlauch (26) abgibt, wenn der Detektordraht (34) unversehrt ist.

3. Sauerstoffgerät nach einem der vorstehenden Ansprüche, **dadurch gekennzeichnet, dass** der Detektordraht (34) in eine Wandung (32) des Atemluftschlauchs (26) einextrudiert ist.

4. Sauerstoffgerät nach einem der vorstehenden Ansprüche, **dadurch gekennzeichnet, dass** es ein Gerät für die häusliche Sauerstofftherapie ist.

5. Sauerstoffgerät nach einem der vorstehenden Ansprüche, **dadurch gekennzeichnet, dass** das Ventil (22) manuell in die Durchgangs-Schaltstellung, in der die Atemgasquelle (14) mit dem Atemluftschlauch (26) verbunden ist, bringbar ist, wenn der Detektordraht (34) durch einen brennenden Atemluftschlauch (26) beeinflusst ist.

6. Sauerstoffgerät nach einem der vorstehenden Ansprüche, **gekennzeichnet durch** eine elektrische Steuereinheit, die eingerichtet ist zum automatischen Durchführen eines Verfahrens mit den Schritten:
(i) Erfassen, ob sich der Widerstand (R) des Detektordrahts (34) so verändert, dass auf einen Brand des Atemluftschlauchs (26) geschlossen werden kann, und
(ii) bejahendenfalls Abgeben eines Warnsignals, insbesondere eines Notrufsignals.

7. Sauerstoffgerät nach einem der vorstehenden Ansprüche, **gekennzeichnet durch** eine elektrische Steuereinheit, die eingerichtet ist zum automatischen Durchführen eines Verfahrens mit den Schritten:
(i) Erfassen, ob der Schmelzdraht (34) ununterbrochen ist und
(ii) bejahendenfalls Bringen oder Halten des Ventils (22) in einer Stellung, in der Atemgas (12) in den Atemluftschlauch (26) abgegeben wird.

## Claims

1. Oxygen apparatus for emitting an oxygen-enriched breathing gas (12) with
(a) a breathing gas source (14) that is configured to produce the oxygen-enriched breathing gas (12) and
(b) a breathing air tube (26), which is configured to guide the breathing gas (12) from the breathing gas source (14) to a point of consumption (36),
**characterised by** the fact that
(c) the breathing air tube (26) comprises a detector wire (34) that is designed as a fuse wire and composed of a detector wire material, which is selected such that the detector wire (34) melts at least locally when the breathing air tube (26) burns, thereby locally destroying the detector wire (34) and causing an increase in electrical resistance (R) by at least one order of magnitude, and
(d) the oxygen apparatus (10) is designed such that the breathing gas source (14) only emits breathing gas (12) into the breathing air tube (26) when the detector wire (34) is intact and configured to interrupt the emission of the breathing gas into the breathing air tube if the detector wire is locally destroyed.

2. Oxygen apparatus according to claim 1, **characterised by** a valve (22), especially a diverter valve
- that is connected to the breathing gas source (14) and the breathing air tube (26), and
- is interconnected with the detector wire (34) such that the valve (22) only emits breathing gas (12) into the breathing air tube (26) when the detector wire (34) is intact.

3. Oxygen apparatus according to one of the above claims, **characterised by** the fact that the detector wire (34) is extruded into a wall (32) of the breathing air tube (26).

4. Oxygen apparatus according to one of the above claims, **characterised by** the fact that it is an apparatus for home oxygen therapy.

5. Oxygen apparatus according to one of the above claims, **characterised by** the fact that the valve (22) can be manually brought into the through-shift position, in which the breathing gas source (14) is connected with the breathing air tube (26), if the detector wire (34) is influenced by a burning breathing air tube (26).

6. Oxygen apparatus according to one of the above claims, **characterised by** an electric control unit that is configured to automatically carry out a method featuring the steps:
(i) identifying whether the resistance (R) of the detector wire (34) changes in such a way that it can be attributed to a burning of the breathing air tube (26), and
(ii) if this transpires to be the case, emitting a warning signal, in particular an emergency signal.

7. Oxygen apparatus according to one of the above claims, **characterised by** an electric control unit that is configured to automatically carry out a method featuring the steps:
(i) identifying whether the fuse wire (34) is uninterrupted and
(ii) if this transpires to be the case, bringing or keeping the valve (22) in a position in which the breathing gas (12) is emitted into the breathing air tube (26).

## Revendications

1. Appareil à oxygène pour distribuer un gaz de respiration (12) enrichi en oxygène, comportant
(a) une source de gaz de respiration (14) qui est conçue pour générer un gaz de respiration (12) enrichi en oxygène, et
(b) un tuyau à air de respiration (26) qui est réalisé pour conduire le gaz de respiration (12) depuis la source de gaz de respiration (14) jusqu'à un lieu de consommation (36),
**caractérisé en ce que**
(c) le tuyau à air de respiration (26) comprend un fil détecteur (34) qui est réalisé sous forme de fil fusible et qui est constitué en un matériau de fil détecteur choisi de telle sorte que le fil détecteur (34) fond au moins localement lorsque le tuyau à air de respiration (26) brûle, de sorte que le fil détecteur (34) est localement détruit et que la résistance électrique (R) augmente d'au moins un ordre de grandeur, et
(d) l'appareil à oxygène (10) est réalisé de telle sorte que la source de gaz de respiration (14) ne distribue du gaz de respiration (12) jusque dans le tuyau à air de respiration (26) que lorsque le fil détecteur (34) est intact, et il est réalisé pour interrompre la distribution du gaz de respiration dans le tuyau à air de respiration lorsque le fil détecteur est localement détruit.

2. Appareil à oxygène selon la revendication 1, **caractérisé par** une vanne (22), en particulier par une vanne de dérivation,
- qui est reliée à la source de gaz de respiration (14) et au tuyau à air de respiration (26), et
- qui est connectée au fil détecteur (34) de telle sorte que la vanne (22) ne distribue du gaz de respiration (12) jusque dans le tuyau à air de respiration (26) que lorsque le fil détecteur (34) est intact.

3. Appareil à oxygène selon l'une des revendications précédentes, **caractérisé en ce que** le fil détecteur (34) est intégré par extrusion dans une paroi (32) du tuyau à air de respiration (26).

4. Appareil à oxygène selon l'une des revendications précédentes, **caractérisé en ce qu'**il est un appareil pour la thérapie domestique à l'oxygène.

5. Appareil à oxygène selon l'une des revendications précédentes, **caractérisé en ce que** la vanne (22) peut être amenée manuellement jusque dans la position de commutation de passage dans laquelle la source de gaz de respiration (14) est reliée au tuyau à air de respiration (26), lorsque le fil détecteur (34) est influencé par un tuyau à air de respiration (26) qui brûle.

6. Appareil à oxygène selon l'une des revendications précédentes, **caractérisé par** une unité de commande électrique qui est conçue pour la mise en oeuvre automatique d'un procédé comprenant les étapes consistant à :
(i) détecter si la résistance (R) du fil détecteur (34) se modifie de manière à pouvoir en déduire un incendie du tuyau à air de respiration (26), et
(ii) si oui, émettre un signal d'alerte, en particulier un signal d'appel de secours.

7. Appareil à oxygène selon l'une des revendications précédentes, **caractérisé par** une unité de commande électrique qui est conçue pour la mise en oeuvre automatique d'un procédé comprenant les étapes consistant à :
(i) détecter si le fil fusible (34) est ininterrompu, et
(ii) si oui, amener ou maintenir la vanne (22) dans une position dans laquelle du gaz de respiration (12) est distribué dans le tuyau à air de respiration (26).
